# EUROPEAN PATENT SPECIFICATION

(11) **EP 4 329 578 B1**
(45) Date of publication and mention of the grant of the patent: **28.01.2026**
(21) Application number: 22724782.2
(22) Date of filing: 25.04.2022
(51) Int. Cl.: A61B 1/00, A61B 1/005

(54) **IMAGE-BASED FUNCTION ACTIVATION IN A MEDICAL VISUALISATION SYSTEM**
BILDBASIERTE FUNKTIONSAKTIVIERUNG IN EINEM MEDIZINISCHEN VISUALISIERUNGSSYSTEM
ACTIVATION DE FONCTION BASÉE SUR L'IMAGE DANS UN SYSTÈME DE VISUALISATION MÉDICALE

(30) Priority: 29.04.2021 DK PA202170195
(43) Date of publication of application: 06.03.2024
(73) Proprietor: Ambu A/S, 2750 Ballerup (DK)
(72) Inventor: NIELSEN, Brian, 2750 Ballerup (DK); SCHRØDER, Morten Rudkjær, 2750 Ballerup (DK)
(74) Representative: COPA Copenhagen Patents
(86) International application number: PCT/EP2022/060912
(87) International publication number: WO 2022/229108

(56) References cited:
- EP-A1- 2 996 541
- EP-A1- 3 777 646
- EP-A2- 2 266 452
- US-A1- 2013 057 713

## Description

The present disclosure relates to a medical visualisation system, a monitor device for a medical visualisation system and an associated method for controlling one or more functions of the medical visualisation system, such as of the monitor device. Particularly, the present disclosure relates to activation of functions of the medical visualisation system based on movement patterns determined based on series of image frames.

### BACKGROUND

For medical visualisation devices, such as endoscopes, when an operator operating such device both hands may be occupied using and controlling the device. Thus, to activate functions, such as capturing of pictures or videos, the operator may need to let go of one hand or have an assisting person operate controls of an associated monitor device. In case the operator removes one hand from the device, the result may be that the visualisation device, e.g. a camera of the visualisation device, moves and is no longer in the desired position.

One suggested solution might be to add a button on a handle of the visualisation device. However, this makes single use visualisation devices more expensive, and require replacement of existing visualisation devices.

EP 3 777 646 A1 discloses an endoscopy display system comprising an endoscope comprising a distal tip, wherein said distal tip has at least two image capturing components, and at least one actuator that, when activated, generates one or more video processing commands, a controller external to said endoscope, wherein said controller comprises a video processing system adapted to transmit commands to said at least two image capturing components and receive an image feed from each of said at least two image capturing components, wherein the video processing system is configured to process the image feeds to combine them into a single, panoramic image feed, and a display system, wherein said display system comprises at least one monitor that concurrently receives the panoramic image feed from the video processing system, and visually displays the panoramic image feed, and wherein said video processing system is adapted to process the panoramic image feed in accordance with the one or more video processing commands to cause at least one of a position of the panoramic image feed on the at least one monitor to change, a zoom into the panoramic image feed, a recording of the panoramic image feed, a freezing of the panoramic image feed, a highlighting the panoramic image feed, or a superimposing of a navigation indicator on the panoramic image feed.

### SUMMARY

It is an object of the present disclosure to provide a solution which at least improves the solutions of the prior art. Thus, the present disclosure relates to a medical visualisation system, a monitor device for a medical visualisation system and an associated method for controlling one or more functions of the medical visualisation system, such as of the monitor device. The method is not part of the claimed invention.

Accordingly, a method for controlling one or more functions in a medical visualisation system is disclosed. Also the medical visualisation system and a monitor device for such medical visualisation system are disclosed.

The medical visualisation system comprises a visualisation device having an insertion cord extending from a proximal cord end to a distal cord end. The visualisation device further comprises an image sensor configured to generate image data indicative of a view at the distal cord end of the insertion cord. A distal portion of the insertion cord may be bendable.

The monitor device comprises a processing unit, such as a GPU, CPU, FPGA or another processing unit known in the art. The monitor device is connectable to the visualisation device of the medical visualisation system, and operable to receive the image data as the image data is being generated by the image sensor. The medical visualisation system may comprise the monitor device. The monitor device may be connectable to the visualisation device by a wired connection. Alternatively or additionally, the monitor device may be connectable to the visualisation device by use of a wireless connection.

The medical visualisation system may comprise a display. For example, the monitor device may comprise a display, such as a touch sensitive display. Alternatively or additionally, the monitor device may be connectable to an external display, such as an external display of the medical visualisation system. The plurality of image frames may be displayed on the display of the medical visualisation system, such as the display of the monitor device or the external display. For example, a live view of the image frames captured by the image sensor may be displayed on the display.

The visualisation device may comprise a handle at the proximal cord end. The handle may comprise an input mechanism, e.g. adapted to receive an input, such as a touch input, in one or more input directions. The input in the one or more input directions may cause a distal portion, e.g. including the distal cord end, of the insertion cord to bend in a corresponding bending direction.

The method comprises receiving a plurality of image frames captured by the image sensor including a first series of image frames, determining based on the first series of image frames a movement pattern of the distal cord end of the insertion cord, determining whether the movement pattern satisfies one or more movement criteria, and in accordance with the movement pattern satisfying a first movement criterion of the one or more movement criteria activating a first function of the medical visualisation system. For example, the processing unit may be operable to receive the first series of image frames captured by the image sensor, determine based on the first series of image frames the movement pattern of the distal cord end of the insertion cord and whether the movement pattern satisfies one or more movement criteria, and in accordance with the movement pattern satisfying a first movement criterion of the one or more movement criteria activate the first function of the medical visualisation system.

Determining the movement pattern based on the first series of image frames may be performed concurrently with receiving the plurality of image frames. For example, an evaluation of the movement pattern may be continuously performed while receiving new image frames captured by the image sensor. Thereby, more complex movement patterns may be used to activate functionality, without significantly influencing the time before the function is activated.

Different movements may activate different functions. Thus, the user may activate a plurality of functions by movement patterns of the distal cord end. For example, in accordance with the movement pattern satisfying a second movement criterion of the one or more movement criteria a second function of the medical visualisation system may be activated. The second movement criterion may be different from the first movement criterion. The second function may be different than the first function.

The first function or the second function may be a capture image function, e.g. saving a frame received from the image sensor in memory of the medical visualisation system. The memory may be a memory of the monitor device and/or the memory may be a remote memory, e.g. located on a server. Alternatively or additionally, the first function or the second function may be starting or stopping a recording of the image data. Alternatively or additionally, the first function or the second function may be activating a filter, changing contrast of the image frames captured by the image sensor, or changing a zoom factor of the image frames captured by the image sensor.

The first function or the second function may be a video capture function comprising a start video capture function and a stop video capture function. The start video capture function and the stop video capture function may be alternately activated.

A visual indication, e.g. a symbol or an alert message, may be displayed, e.g. on the display of the medical visualisation system, such as the display of the monitor device and/or an external display coupled to the monitor device, when the first function and/or the second function is activated, such as in response to the first function and/or the second function being activated. For example, the monitor device may be configured to cause display of the visual indication. The visual indication aids the user to recognise the function having been activated and confirms to the user that the intended function was activated. For example, the user is alerted in case he mistakenly captured a still image, when the intention was to start a video recording.

The first movement criterion and/or the second movement criterion may include holding the distal cord end still for a predetermined time period. Alternatively or additionally, the first movement criterion and/or the second movement criterion may include moving the distal cord end up, e.g. followed by moving the distal cord end down, optionally followed by holding the distal cord end still for a second predetermined time period. Alternatively or additionally, the first movement criterion and/or the second movement criterion may include moving the distal cord end to a first side, e.g. followed by moving the distal cord end to a second side opposite the first side, optionally followed by holding the distal cord end still for a third predetermined time period. The predetermined time period, the second predetermined time period and/or the third predetermined time period may be between 0.5-10 seconds, such as between 1-6 seconds, such as 6 seconds, 4 seconds or 3 seconds, or such as between 0.8-1.5 seconds. The predetermined time period, the second predetermined time period and/or the third predetermined time period may be user adjustable, e.g. in a settings menu. Alternatively or additionally, the predetermined time period, the second predetermined time period and/or the third predetermined time period may be set by a learning procedure, wherein the user performs the desired movement pattern, e.g. a plurality of times, as he/she desires, and the respective time period may be set in accordance with the time period the user has held the distal cord end still during the performed movement pattern. In case of the user repeating the desired movement pattern a plurality of times, the respective time period may be set to a mean of the time period for which the user has held the distal cord end still during each of the performances of the desired movement pattern.

The determination of the movement pattern of the distal cord end may be based on software image processing, such as feature detection or motion vector.

Determining the movement pattern of the distal cord end may be achieved by comparing image features between succeeding image frames in the first series of image frames. The processing unit may be configured to determine the movement pattern of the distal cord end by comparing image features between succeeding image frames in the first series of image frames. For example, determining the movement pattern of the distal cord end may be achieved by comparing image features for each image frame in the first series of image frames with a subsequent or previous image frame in the first series of image frames. The image features of the image frames may comprise corners, SIFT (scale-invariant feature transform), SURF (speeded up robust features), blobs, edges and/or pixel colour information.

As an example, a determination that the distal cord end is held still may be based upon the comparison of the image features, wherein it is determined that the distal cord end is held still if a difference between image features of image frames in the first series of image frames are less than a pre-set threshold.

As another example, a determination that the distal cord end is moved in a particular direction (e.g. up, down, left or right) may be based upon the comparison of the image features, wherein it is determined that the distal cord end is moved in the particular direction if differences between image features of image frames in the first series of image frames have similar magnitude.

The present disclosure provides a solution which allows an operator of the visualisation device to activate functionality without having to remove a hand from the visualisation device or having to ask an assistant to activate the function. The present disclosure is further advantageous in that it may be realised by a software upgrade rather than having to replace existing or stocked visualisation devices.

The invention is defined in claim 1.

### BRIEF DESCRIPTION OF THE FIGURES

Embodiments of the disclosure will be described in more detail in the following with regard to the accompanying figures. The figures show one way of implementing the present disclosure and are not to be construed as being limiting to other possible embodiments falling within the scope of the attached claim set.
Fig. 1 is a schematic diagram illustrating an exemplary medical visualisation system,
Fig. 2 is a block diagram of an exemplary monitor device, and
Figs. 3A-3E illustrate different exemplary movements patterns.

### DETAILED DESCRIPTION

Various exemplary embodiments and details are described hereinafter, with reference to the figures when relevant. It should be noted that the figures may or may not be drawn to scale and that elements of similar structures or functions are represented by like reference numerals throughout the figures. It should also be noted that the figures are only intended to facilitate the description of the embodiments. In addition, an illustrated embodiment needs not have all the aspects or advantages shown. An aspect or an advantage described in conjunction with a particular embodiment is not necessarily limited to that embodiment and can be practiced in any other embodiments even if not so illustrated, or if not so explicitly described.

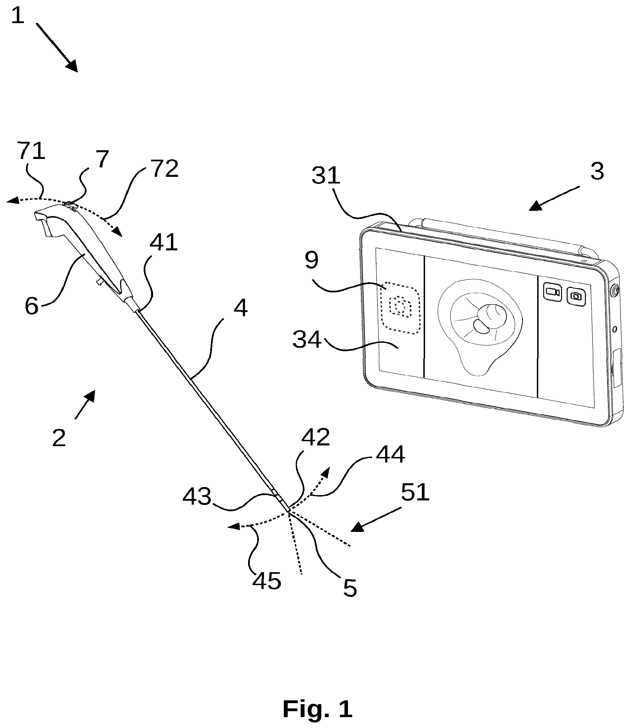

Fig. 1 is a schematic diagram illustrating an exemplary medical visualisation system 1. The medical visualisation system 1 comprises a visualisation device 2 and a monitor device 3. The visualisation device 2 comprises an insertion cord 4 extending from a proximal cord end 41 to a distal cord end 42. The visualisation device 2 comprises an image sensor 5 configured to generate image data indicative of a view 51 from the distal cord end 42 of the insertion cord 4. The image sensor 5 may be a camera located within the insertion cord 4 at the distal cord end pointing in an outward direction to generate the desired view 51. In other exemplary visualisation devices according to the present disclosure, the image sensor 5 may be located at a distance from the distal cord end, e.g. in the handle 6, and optical elements, e.g. optical fibres, may provide a visual connection between the image sensor 5 and the view 51 from the distal cord end 42.

A distal portion 43 of the insertion cord 4 is bendable, such that the field of view of the image sensor 5 can be changed. For example, as illustrated the distal portion 43 is bendable in a first bending direction 44 and in a second bending direction 45.

The visualisation device may comprise a handle 6 at the proximal cord end. The handle 6 may comprise an input mechanism 7 adapted to receive an input, such as a touch input, in one or more input directions, such as in a first input direction 71 and a second input direction 72, as illustrated. The touch input on the input mechanism 7 causes the distal portion 43 of the insertion cord 4 to bend in a corresponding bending direction. For example a touch input in the first input direction 71 causes the distal portion 43 to bend in the first bending direction 44. A touch input in the second input direction 72 causes the distal portion 43 to bend in the second bending direction 45. A user is therefore able to bend the insertion cord 4 by use of the input mechanism 7.

The monitor device 3 is connectable to the visualisation device 2. For example, the visualisation device 2 may be connected with a cable to an input port on the monitor device 3. Alternatively, the visualisation device 2 may be wirelessly connected to the monitor device 3. The monitor device 3 is operable to receive the image data from the visualisation device 2 as the image data is being generated by the image sensor 5. The monitor device may comprise a display 34 as illustrated, to display the received image data. However, in other exemplary medical visualisation systems, the monitor device 3 may be connected to an external display, and the monitor device 3 may cause display of the received image data on the external display. Thus, the monitor device 3 may be configured to receive real-time data from the visualisation device 2.

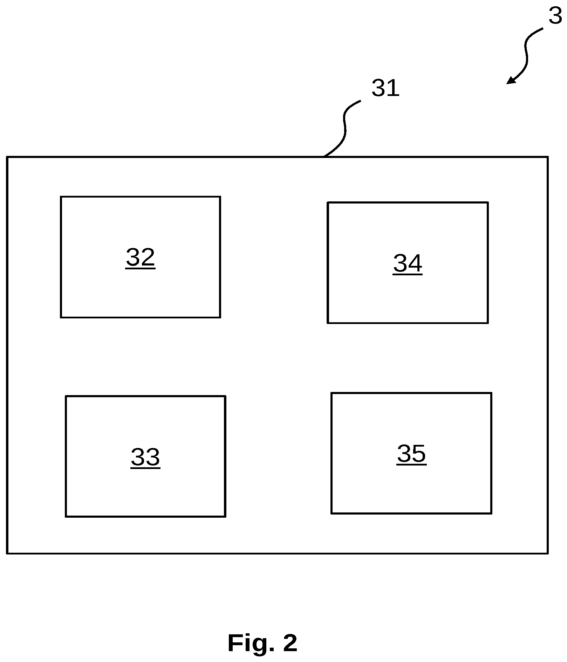

Fig. 2 is a block diagram of an exemplary monitor device 3, such as the monitor device 3 as illustrated in Fig. 1. The monitor device 3 comprises a processing unit 32. The processing unit 32 may be operable to receive the image data as the image data is being generated by the image sensor 5. Furthermore, the monitor device 3 may comprise memory 33 and/or a display 34 as illustrated. The monitor device 3 further comprises a device communication interface 35 for connecting the visualisation device 2 and the monitor device 3. For example, the device communication interface 35 may comprise a socket for receiving a plug of a cable of the visualisation device 2. Alternatively or additionally, the device communication interface 35 may comprise a wireless communication unit for wirelessly communicating with a corresponding wireless communication unit of the visualisation device 2. The monitor device 3 may comprise a housing 31. The housing 31 may comprise the components of the monitor device 3, such as the processing unit 32, the memory 33, and/or the display 34 and/or the device communication interface 35.

The processing unit 32 of the monitor device 3, may receive a first series of image frames captured by the image sensor. This first series of image frame can be a continuously changing real-time series of image frames, such that the content of the series may be continuously changing.

Based upon the first series of image frames a movement pattern that is representative of any movement (or lack thereof) of the distal end of the insertion cord may be determined, e.g. by the processing unit 32. It may further be determined, e.g. by the processing unit 32, whether the determined movement pattern satisfies one or more movement criteria. If the movement pattern satisfies one of the one or more movement criteria, a function, e.g. corresponding to the satisfied criterion, that is associated with the medical visualisation system, may be activated, e.g. by the processing unit 32. Thus, the functions of the medical visualisation system may be controlled, e.g. by the processing unit 32, based on image based detection of certain movements of the image sensor 5 of the visualisation device 2.

The functions, which may be activated, may be a capture image function causing the processing unit to save a frame received from the image sensor, e.g., in the memory 33 of the monitor device 3 or in an external memory, starting or stopping a recording of the image data to be saved, e.g., in the memory 33 or an external memory, activation or deactivation of a filter, changing of the contrast of image frames captured by the image sensor, or change of a zoom factor of the image frames captured by the image sensor 5. The functions may be functions of the visualisation device 2, monitor device 3 and/or the medical visualisation system 1 in general.

Different functions of the visualisation device 2 may have associated to them different movement criteria, such that a user can activate a number of different functions by moving the distal cord end 42 in pre-defined movement patterns. A movement pattern may satisfy a first movement criteria that activates a first function of the medical visualisation system. A movement pattern may satisfy a second movement criteria that activates a second function of the medical visualisation system.

The movement pattern of the distal cord end 42 can come from a number of different sources. It may come from bending of the bendable portion 43 of the insertion cord but may alternatively or additionally come from movement of the whole of the insertion cord 4, e.g., caused by the user rotating the entire visualisation device 2. The present disclosure allows a user of the medical visualisation system 1 to move the distal cord end 42 relative to the environment and based on the movement activate one or more functions of the medical visualisation system 1.

The movement pattern may correspond to the movement of the distal cord end 42 of the insertion cord 4 and a movement pattern could consist of a sequence of different movements, such as holding the distal cord end 42 of the insertion cord 4 still, moving the distal cord end 42 in a predefined direction or rotating it, or a combination thereof. The movement criteria to be satisfied for the activation of a function may be a specific sequence of movements. For example, an exemplary movement criteria may include holding the distal cord end 42 still for a predetermined time period, moving the distal cord end 42 up followed by moving the distal cord end 42 down, optionally followed by holding the distal cord end 42 still for a second predetermined time period, Another exemplary movement criteria may include moving the distal cord end 42 to a first side followed by moving the distal cord end 42 to a second side opposite the first side, optionally followed by holding the distal cord end 42 still for a third predetermined time period.

The movement criteria may be composed of a finite number of movements in a specified sequence order and/or for a specified amount of time, either as a whole for all the movements or for individual movements. The movement criteria could therefore be as short as 1-2 movements to as long as 6-7 movements or more. The predetermined time period, the second predetermined time period and/or the third predetermined time period could be between 0.5-10 seconds, such as between 1-6 seconds, such as 6 seconds, 4 seconds or 3 seconds, or such as between 0.8-1.5 seconds. In some examples, the time periods may be user defined. Generally, the time periods may be different. For example, an intermediate holding still criteria may be shorter than an end of sequence hold. Holding the distal cord end 42 still could also be used to distinguish between different movements, which may allow for exact separation between movements.

Some movement criteria may alternately activate and deactivate a similar functionality. For example the same movement pattern may start and stop a video capture function, based on whether the video capture function is activated or not.

Different functions could also have different movement criteria lengths. For example, some functions may have a criteria that is composed of 2-3 different movements and some may have 5-6 different movements in the criteria. The length and/or the start and/or the end of the first series of image frames may be dynamically determined based upon the sequences of movements. In some embodiments, the start of the first series of image frames may be determined based on a characteristic movement, such as holding the distal cord end 42 still, e.g. for 3 seconds. An end of the first series of image frames may be determined based on the same or another characteristic movement. In some embodiments, received image frames may be continuously evaluated to determine whether a first series of image frames is indicative of a movement pattern satisfying a movement criteria.

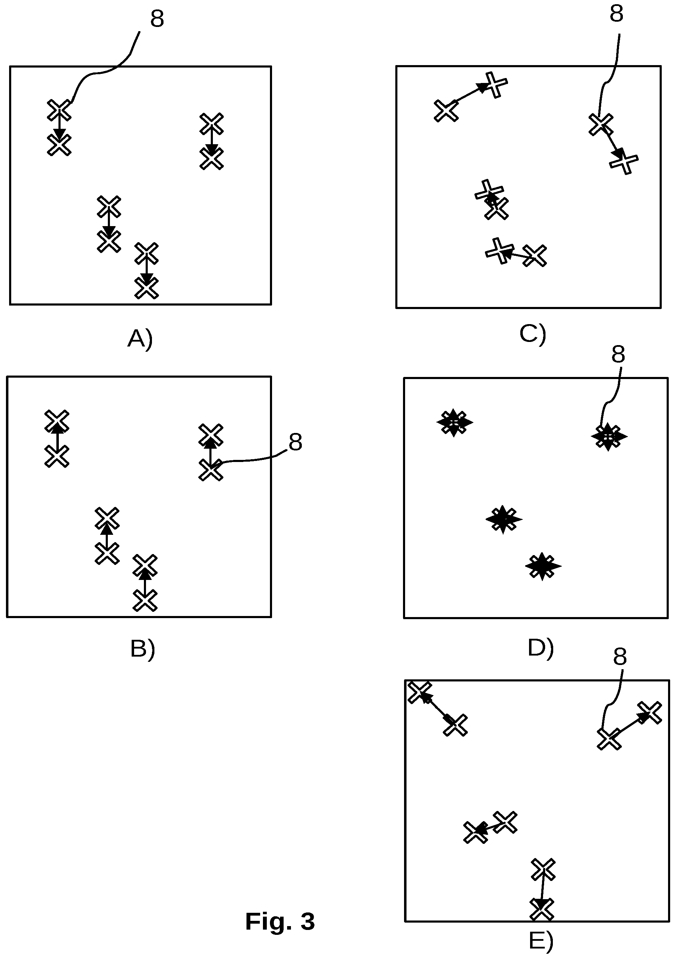

Determining the movement pattern of the distal cord end 42 may be achieved by comparing image features between image frames in the first series of images. For example, by comparing image features for each image frame in the first series of image frames with a subsequent or previous image frame in the first series of image frames. The determination of the movement pattern of the distal end could be based on software image processing, such as feature detection or motion vector. For example, as schematically illustrated in Figs. 3A-3E, image features 8 (indicated by X's) may be determined, e.g. by the processing unit 32, and their movement, as indicated by arrows may be tracked.

In Figs. 3A-3E, five different kind of exemplary movements are illustrated. In Fig. 3A an up movement of the distal cord end 42 is illustrated, e.g. caused by bending of the distal portion 43 of the insertion cord 4 in a first bending direction 44, causing the image features 8 to collectively move down. In Fig. 3B a down movement of the distal cord end 42 is illustrated, e.g. caused by bending of the distal portion 43 of the insertion cord 4 in a second bending direction 45, causing the image features 8 to collectively move up. In Fig. 3C a counter-clockwise rotation of the distal cord end 42 is illustrated, e.g. caused by counter-clockwise rotation of the entire visualisation device 2, causing the image features 8 to move in a clockwise manner. In Fig. 3D absence or very little movement, i.e. the user holding the visualisation device 2 still, is illustrated. As illustrated in Fig. 3D, holding still the distal cord end 42 may entail small movements in all directions, e.g. due to minor movements of the hands of the operator, external forces acting on the distal cord end 42, e.g. due to the patient breathing, or similar. In Fig. 3E an example of a movement of the distal cord end 42 advancing through an opening, such as when advancing through an intestine, causing the image features 8 to move outwards.

As illustrated in Figs. 3A-3E, the movement pattern of the distal cord end 42 may be determined, e.g. by the processing unit 32 of the monitor device 3, by comparing image features for each image frame in a first series of image frames with a subsequent or previous image frame in the first series of image frames. The image features of the image frames may comprise corners, SIFT, SURF, Blobs, edges and/or pixel colour information.

The determination that the distal end is held still may be based upon comparison of image features. For example, it may be determined that the distal end is held still if a difference between image features of image frames in the first series of image frames are less than a pre-set threshold.

A visual indicator, e.g. a symbol or an alert message, may be displayed when a function, such as the first function and/or the second function is activated. For example, the monitor device 3, as illustrated in Fig. 1, may display a visual indicator 9, such as an image or video capture symbol, indicating that a corresponding function has been activated. For example, the visual indicator 9 may be flashing when the corresponding function is activated. For example, when the function is saving an image frame, the visual indicator 9 may be a temporary showing of a camera icon. Alternatively or additionally, when the function starts or stops video capture, the visual indicator 9 may be showing a video camera icon.

The monitor device 3 may further aid the user in finding and/or teaching the correct movement criteria for specific functions by showing, e.g. on the display 34, the most popular functions and their corresponding movement criteria for activating them. For example, the monitor device may show animations of the most popular functions together with a text of the associated function. Alternatively, the monitor device may show a textual guide of the movements necessary for activating an associated function.

The disclosure has been described with reference to a preferred embodiment. However, the endoscope may be a rigid endoscope, e.g. a laparoscope, or a flexible endoscope adapted for bending of the distal end left-right and up-down, e.g. a gastroscope. The endoscope may be reusable, but preferably the endoscope is intended for single use.

Throughout the description, the use of the terms "first", "second", "third", "fourth", "primary", "secondary", "tertiary" etc. does not imply any particular order or importance but are included to identify individual elements. Furthermore, the labelling of a first element does not imply the presence of a second element and vice versa.

### LIST OF REFERENCES

- 1: medical visualisation system
- 2: visualisation device
- 3: monitor device
- 4: insertion cord
- 5: image sensor
- 6: handle
- 7: input mechanism
- 8: image features
- 9: visual indicator
- 31: housing
- 32: processing unit
- 33: memory
- 34: display
- 35: device communication interface
- 41: proximal cord end
- 42: distal cord end
- 43: distal portion
- 44: first bending direction
- 45: second bending direction
- 51: view
- 71: first input direction
- 72: second input direction

## Claims

1. A medical visualisation system (1) comprising a visualisation device (2) and a monitor device (3),
wherein the visualisation device comprises an insertion cord (4) extending from a proximal cord end to a distal cord end, and comprising an image sensor (5) configured to generate image data indicative of a view at the distal cord end of the insertion cord,
wherein the monitor device comprises a processing unit (32), the monitor device being connectable to the visualisation device and operable to receive the image data as the image data is being generated by the image sensor,
**characterised in that** the processing unit is operable to receive a first series of image frames captured by the image sensor, determine based on the first series of image frames a movement pattern of the distal cord end of the insertion cord and whether the movement pattern satisfies one or more movement criteria, and in accordance with the movement pattern satisfying a first movement criterion of the one or more movement criteria activate a first function of the medical visualisation system.

2. Medical visualisation system according to claim 1, wherein the visualisation device comprises a handle at the proximal cord end, the handle comprises an input mechanism adapted to receive an input, such as a touch input, in one or more input directions, and wherein the input in the one or more input directions causes a distal portion of the insertion cord to bend in a corresponding bending direction.

3. Medical visualisation system according to any one of the preceding claims, wherein the processing unit is operable to activate a second function in accordance with the movement pattern satisfying a second movement criterion of the one or more movement criteria.

4. Medical visualisation system according to any one of the preceding claims, wherein the first function or the second function is a capture image function causing the processing unit to save a frame received from the image sensor in a memory of the monitor system, starting or stopping a recording of the image data to be saved in the memory, activation or deactivation of a filter, change of contrast of image frames captured by the image sensor, or change of a zoom factor of the image frames captured by the image sensor.

5. Medical visualisation system according to any one of the preceding claims, wherein the first function or the second function is a video capture function comprising a start video capture function and a stop video capture function, wherein the start video capture function and the stop video capture function is alternately activated.

6. Medical visualisation system according to any one of the preceding claims, wherein the monitor device is further configured to, in response to the first function and/or the second function being activated, cause display of a visual indication, e.g. a symbol or alert message, on a display.

7. Medical visualisation system according to any one of the preceding claims, wherein the first movement criterion includes holding the distal cord end still for a predetermined time period, moving the distal cord end up followed by moving the distal cord end down optionally followed by holding the distal cord end still for a second predetermined time period, or moving the distal cord end to a first side followed by moving the distal cord end to a second side opposite the first side optionally followed by holding the distal cord end still for a third predetermined time period.

8. Medical visualisation system according to any one of the preceding claims, wherein the processing unit is configured to determine the movement pattern of the distal cord end by comparing image features for each image frame in the first series of image frames with a subsequent or previous image frame in the first series of image frames.

9. Medical visualisation system according to claim 8 when dependent on claim 7, wherein the processing unit is configured to determine that the distal cord end is held still is based upon the comparison of the image features, wherein it is determined that the distal cord end is held still if the difference between image features of image frames in the first series of image frames are less than a pre-set threshold.

10. Medical visualisation system according to any one of the preceding claims, wherein the processing unit is configured to determine the movement pattern of the distal cord end based upon software image processing, such as feature detection or motion vector.

11. Medical visualisation system according to any one of the preceding claims, wherein the processing unit is a GPU or CPU.

## Patentansprüche

1. Medizinisches Visualisierungssystem (1), umfassend eine Visualisierungsvorrichtung (2) und eine Überwachungsvorrichtung (3),
wobei die Visualisierungsvorrichtung einen Einführstrang (4) umfasst, der sich von einem proximalen Strangende zu einem distalen Strangende erstreckt, und umfassend einen Bildsensor (5), der dazu konfiguriert ist, Bilddaten zu erzeugen, die eine Ansicht am distalen Strangende des Einführstrangs angeben,
wobei die Überwachungsvorrichtung eine Verarbeitungseinheit (32) umfasst, wobei die Überwachungsvorrichtung mit der Visualisierungsvorrichtung verbindbar ist und betreibbar ist, um die Bilddaten zu empfangen, wenn die Bilddaten vom Bildsensor erzeugt werden,
**dadurch gekennzeichnet, dass** die Verarbeitungseinheit betreibbar ist, um eine erste Serie von Bildrahmen, die vom Bildsensor aufgenommen wurden, zu empfangen, um, basierend auf der ersten Serie von Bildrahmen ein Bewegungsmuster des distalen Strangendes des Einführstrangs zu bestimmen und um zu bestimmen, ob das Bewegungsmuster ein oder mehrere Bewegungskriterien erfüllt, und um, in Übereinstimmung mit dem Bewegungsmuster, das ein erstes Bewegungskriterium des einen oder der mehreren Bewegungskriterien erfüllt, eine erste Funktion des medizinischen Visualisierungssystems zu aktivieren.

2. Medizinisches Visualisierungssystem nach Anspruch 1, wobei die Visualisierungsvorrichtung einen Griff am proximalen Strangende umfasst, wobei der Griff einen Eingabemechanismus umfasst, der dazu angepasst ist, eine Eingabe, wie eine Berührungseingabe, in einer oder mehreren Eingaberichtungen zu empfangen, und wobei die Eingabe in der einen oder den mehreren Eingaberichtungen bewirkt, dass sich ein distaler Abschnitt des Einführstrangs in einer entsprechenden Biegerichtung biegt.

3. Medizinisches Visualisierungssystem nach einem der vorstehenden Ansprüche, wobei die Verarbeitungseinheit betreibbar ist, um in Übereinstimmung mit dem Bewegungsmuster, das ein zweites Bewegungskriterium des einen oder der mehreren Bewegungskriterien erfüllt, eine zweite Funktion zu aktivieren.

4. Medizinisches Visualisierungssystem nach einem der vorstehenden Ansprüche, wobei die erste Funktion oder die zweite Funktion eine Bildaufnahmefunktion ist, die die Verarbeitungseinheit dazu veranlasst, ein vom Bildsensor empfangenes Bild in einem Speicher des Überwachungssystems zu speichern, eine Aufzeichnung der im Speicher zu speichernden Bilddaten zu starten oder zu stoppen, einen Filter zu aktivieren oder zu deaktivieren, den Kontrast der vom Bildsensor aufgenommenen Bildrahmen zu ändern oder den Zoomfaktor der vom Bildsensor aufgenommenen Bildrahmen zu ändern.

5. Medizinisches Visualisierungssystem nach einem der vorstehenden Ansprüche, wobei die erste Funktion oder die zweite Funktion eine Videoaufnahmefunktion ist, umfassend eine Videoaufnahme-Startfunktion und eine Videoaufnahme-Stoppfunktion, wobei die Videoaufnahme-Startfunktion und die Videoaufnahme-Stoppfunktion abwechselnd aktiviert werden.

6. Medizinisches Visualisierungssystem nach einem der vorstehenden Ansprüche, wobei die Überwachungsvorrichtung weiter dazu konfiguriert ist, als Reaktion auf die Aktivierung der ersten Funktion und/oder der zweiten Funktion eine Anzeige einer visuellen Angabe, z. B. eines Symbols oder einer Warnmeldung, auf einem Display zu bewirken.

7. Medizinisches Visualisierungssystem nach einem der vorstehenden Ansprüche, wobei das erste Bewegungskriterium einschließt ein Stillhalten des distalen Strangendes für einen vorbestimmten Zeitraum, ein Bewegen des distalen Strangendes nach oben, gefolgt von einem Bewegen des distalen Strangendes nach unten, optional, gefolgt von einem Stillhalten des distalen Strangendes für einen zweiten vorbestimmten Zeitraum, oder einem Bewegen des distalen Strangendes zu einer ersten Seite, gefolgt von einem Bewegen des distalen Strangendes zu einer der ersten Seite gegenüberliegenden zweiten Seite, optional, gefolgt von einem Stillhalten des distalen Strangendes für einen dritten vorbestimmten Zeitraum.

8. Medizinisches Visualisierungssystem nach einem der vorstehenden Ansprüche, wobei die Verarbeitungseinheit dazu konfiguriert ist, das Bewegungsmuster des distalen Strangendes durch Vergleichen von Bildmerkmalen für jeden Bildrahmen in der ersten Serie von Bildrahmen mit einem nachfolgenden oder vorhergehenden Bildrahmen in der ersten Serie von Bildrahmen zu bestimmen.

9. Medizinisches Visualisierungssystem nach Anspruch 8, wenn abhängig von Anspruch 7, wobei die Verarbeitungseinheit dazu konfiguriert ist, basierend auf dem Vergleich der Bildmerkmale zu bestimmen, dass das distale Strangende stillgehalten wird, wobei bestimmt wird, dass das distale Strangende stillgehalten wird, wenn die Differenz zwischen den Bildmerkmalen der Bildrahmen in der ersten Serie von Bildrahmen kleiner als ein voreingestellter Schwellenwert ist.

10. Medizinisches Visualisierungssystem nach einem der vorstehenden Ansprüche, wobei die Verarbeitungseinheit dazu konfiguriert ist, das Bewegungsmuster des distalen Strangendes basierend auf einer Software-Bildverarbeitung, wie einer Merkmalserfassung oder einem Bewegungsvektor, zu bestimmen.

11. Medizinisches Visualisierungssystem nach einem der vorstehenden Ansprüche, wobei die Verarbeitungseinheit eine GPU oder eine CPU ist.

## Revendications

1. Système de visualisation médicale (1) comprenant un dispositif de visualisation (2) et un dispositif de surveillance (3),
dans lequel le dispositif de visualisation comprend un cordon d'insertion (4) s'étendant d'une extrémité proximale de cordon à une extrémité distale de cordon, et comprenant un capteur d'image (5) configuré pour générer des données d'image représentatives d'une vision à l'extrémité distale de cordon du cordon d'insertion,
dans lequel le dispositif de surveillance comprend une unité de traitement (32), le dispositif de surveillance pouvant être connecté au dispositif de visualisation et étant conçu pour recevoir les données d'image au fur et à mesure que les données sont générées par le capteur d'image,
**caractérisé en ce que** l'unité de traitement est conçue pour recevoir une première série de trames d'image capturées par le capteur d'image, déterminer, sur la base de la première série de trames d'image, un motif de mouvement de l'extrémité distale de cordon du cordon d'insertion, et si le motif de mouvement satisfait à un ou plusieurs critères de mouvement, et, lorsque le motif de mouvement satisfait à un premier critère de mouvement parmi les un ou plusieurs critères de mouvement, activer une première fonction du système de visualisation médicale.

2. Système de visualisation médicale selon la revendication 1, dans lequel le dispositif de visualisation comprend une poignée à l'extrémité proximale de cordon, la poignée comprend un mécanisme d'entrée adapté pour recevoir une entrée, telle qu'une entrée tactile, dans une ou plusieurs directions d'entrée, et dans lequel l'entrée dans les une ou plusieurs directions d'entrée entraîne la courbure d'une partie distale du cordon d'insertion dans une direction de courbure correspondante.

3. Système de visualisation médicale selon l'une quelconque des revendications précédentes, dans lequel l'unité de traitement est conçue pour activer une seconde fonction lorsque le motif de mouvement satisfait à un second critère de mouvement parmi les un ou plusieurs critères de mouvement.

4. Système de visualisation médicale selon l'une quelconque des revendications précédentes, dans lequel la première fonction ou la seconde fonction est une fonction de capture d'image qui amène l'unité de traitement à sauvegarder une trame reçue du capteur d'image dans une mémoire du système de surveillance, démarre ou arrête un enregistrement des données d'image à sauvegarder dans la mémoire, une activation ou désactivation d'un filtre, un changement de contraste de trames d'image capturées par le capteur d'image, ou un changement d'un facteur de zoom des trames d'image capturées par le capteur d'image.

5. Système de visualisation médicale selon l'une quelconque des revendications précédentes, dans lequel la première fonction ou la seconde fonction est une fonction de capture vidéo comprenant une fonction de démarrage de capture vidéo et une fonction d'arrêt de capture vidéo, dans lequel la fonction de démarrage de capture vidéo et la fonction d'arrêt de capture vidéo sont activées de manière alternée.

6. Système de visualisation médicale selon l'une quelconque des revendications précédentes, dans lequel le dispositif de surveillance est en outre configuré pour, en réponse à l'activation de la première fonction et/ou de la seconde fonction, provoquer l'affichage d'une indication visuelle, par exemple un symbole ou un message d'alerte, sur un afficheur.

7. Système de visualisation médicale selon l'une quelconque des revendications précédentes, dans lequel le premier critère de mouvement inclut le maintien de l'extrémité distale de cordon immobile pendant une période de temps prédéterminée, le déplacement de l'extrémité distale de cordon vers le haut suivi du déplacement de l'extrémité distale de cordon vers le bas facultativement suivi du maintien de l'extrémité distale de cordon immobile pendant une deuxième période de temps prédéterminée, ou le déplacement de l'extrémité distale de cordon vers un premier côté suivi du déplacement de l'extrémité distale de cordon vers un second côté opposé au premier côté facultativement suivi du maintien de l'extrémité distale de cordon immobile pendant une troisième période de temps prédéterminée.

8. Système de visualisation médicale selon l'une quelconque des revendications précédentes, dans lequel l'unité de traitement est configurée pour déterminer le motif de mouvement de l'extrémité distale de cordon en comparant des caractéristiques d'image pour chaque trame d'image dans la première série de trames d'image à une trame d'image suivante ou précédente dans la première série de trames d'image.

9. Système de visualisation médicale selon la revendication 8 lorsqu'elle dépend de la revendication 7, dans lequel l'unité de traitement est configurée pour déterminer que l'extrémité distale de cordon est maintenue immobile sur la base de la comparaison des caractéristiques d'image, dans lequel il est déterminé que l'extrémité distale de cordon est maintenue immobile si la différence entre des caractéristiques d'image de trames d'image dans la première série de trames d'image est inférieure à un seuil prédéfini.

10. Système de visualisation médicale selon l'une quelconque des revendications précédentes, dans lequel l'unité de traitement est configurée pour déterminer le motif de mouvement de l'extrémité distale de cordon sur la base d'un traitement d'image par logiciel, tel qu'un vecteur de détection de caractéristique ou un vecteur de mouvement.

11. Système de visualisation médicale selon l'une quelconque des revendications précédentes, dans lequel l'unité de traitement est une GPU ou une CPU.
